# EUROPEAN PATENT SPECIFICATION

(11) **EP 1 002 788 B1**
(45) Date of publication and mention of the grant of the patent: **10.03.2004**
(21) Application number: 99307523.3
(22) Date of filing: 23.09.1999
(51) Int. Cl.: C07C 67/343, C07C 69/614

(54) **Process for preparing halogenated phenylmalonates**
Verfahren zur Herstellung halogenierter Phenylmalonaten
Procédé de préparation de malonates de phényle halogénés

(30) Priority: 25.09.1998 US 160695
(43) Date of publication of application: 24.05.2000
(73) Proprietor: BASF AKTIENGESELLSCHAFT, 67056 Ludwigshafen (DE)
(72) Inventor: Meyer, Oliver, 55218 Ingelheim (DE); Eisenacht, Rudi, 55131 Mainz (DE)
(74) Representative: Langfinger, Klaus Dieter, Dr.

(56) References cited:
- SETSUNE, J. ET AL: "Copper(I)-promoted coupling reaction of aryl halides with sodium diethylmalonate" CHEMISTRY LETTERS,1981, pages 367-370, XP000881238

## Description

Halogenated phenylmalonates are useful as intermediates for the preparation of a variety of compounds which are useful as agrochemicals, pharmaceuticals or liquid crystals. In particular, they are key intermediates in the preparation of fungicidal 6-(halophenyl)-triazolopyrimidines.

SETSUNE, J. et.al.: Chemistry Letters, 1981, pages 367-370 disclose a method of coupling phenylhalides with sodium diethylmalonate in the presence of copper(I) salts. However, good yields are obtainable only with phenylbromides substituted with electron withdrawing groups when 1 equivalent of phenylbromide is reacted with 1.2 equivalents of sodium diethylmalonate according to this document. Moreover, use of two equivalents of sodium diethylmalonate gave even lower yields.

The use of five equivalents of sodium diethylmalolanate is suggested in the prior art. Arylbromides substituted by two or more halogen atoms produces undesired side-reactions and decreases the yields.

Therefore, the methods known from the prior art are not entirely satisfactory for large scale production, since the yields of the reactions starting from halogenated phenylbromides are often low.

The present invention provides an effective and efficient process for the preparation of dialkyl phenylmalonates of formula I, 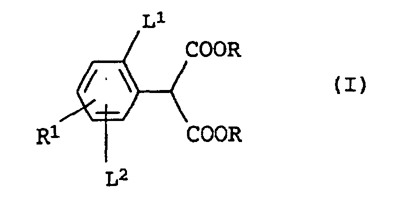 wherein
L¹ and L² each independently represent a fluoro or chloro atom;
R¹ represents a hydrogen or halogen atom or an C₁-C₁₀ alkyl or C₁-C₁₀-alkoxy group; and
R represents an C₁-C₄-alkyl group,
which comprises
treating a 1.0 mole of phenylbromide of formula II 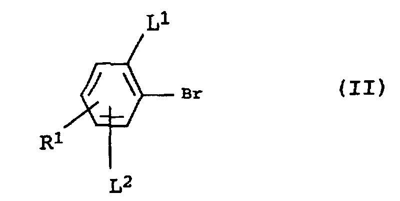 wherein
R¹, L¹ and L² have the meaning given for formula I, with 2.0 to 4.0 moles of a dialkyl malonate of formula III 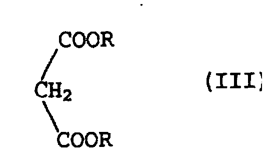 wherein R has the meaning given for formula I,
in an inert solvent in the presence of 2.0 to 3.8 moles of a base and 0.05 to 0.9 moles of copper-(I)bromide.

It is, therefore, an object of the present invention to provide an efficient new process for the preparation of phenylmalonates.

Other objects and advantages of the present invention will be apparent to those skilled in the art from the following description and the appended claims.

### Description

In general terms, unless otherwise stated herein, the term alkyl as used herein with respect to a radical or moiety refer to a straight or branched chain radical or moiety. As a rule, such radicals have up to 10, in particular up to 6 carbon atoms. Suitably an alkyl moiety has from 1 to 6 carbon atoms, preferably from 1 to 3 carbon atoms. A preferred alkyl moiety is the methyl or especially the ethyl group.

Suitable bases are strong bases, preferably alkali metals, such as sodium, alkali hydrides, such as sodium or potassium hydride, alkali amides, such as sodium amide, alkali alkylamides, such as lithium diisopropylamide, alkali alkoxides such as potassium tert.-butoxide, or alkali alkanes, such as butyllithium.

The term "enolate" refers to the deprotonated dialkylmalonate of formula IIIA 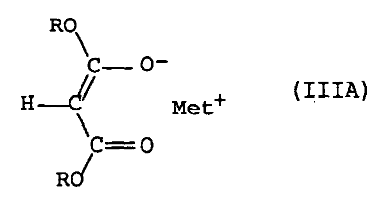 wherein Met represents the metal atom of the base used or copper.

L² is preferably attached in the ortho-position with respect to the bromo atom in formula II. In a particularly preferred embodiment the reaction is carried out with 2-chloro-6-fluorobromobenzene, 2,6-difluorobromobenzene, 2,4,6-trichlorobromobenzene or 2,4,6-trifluorobromobenzene, in particular 2,4,6-trifluorobromobenzene.

Further preferred embodiments of the process according to the present invention is a process wherein:
- the base is sodium hydride;
- 1 mole of phenylbromide of formula II is treated with the enolate obtained from 2.0 to 4.0 moles of dialkyl malonate and 2.0 to 3.8 moles of the base; preferably the dialkyl malonate of formula I is used in excess with respect to the base, the molar ratio of the dialkyl malonate of formula II to the base is preferably in the range of 1 : 1 to 1.5 to 1, in particular in the range of 1.1 : 1 to 1.3 : 1;
- a mixture consisting of the phenylbromide of formula II, the enolate obtained from the dialkyl malonate of formula III, the strong base, a copper salt, optionally a complexing agent and an inert solvent is stirred at temperatures between room temperature and 150°C;
- the inert solvent selected from the group consisting of diethylether, diisopropylether, tert-butylmethylether, 2,2-dimethoxypropane, diethoxyethane, tetrahydrofuran, tetrahydropyran and dioxane or a mixture of these solvents, in particular 1,4-dioxane;
- 0.05 to 0.9 mole of the copper-(I) bromide related to 1 mole of phenylbromide of formula II is used;
- the copper-(I) bromide is complexed by a dialkylsulphide, preferably a di-C₁₋₄-alkylsulphide, in particular dimethylsulphide;
- R represents a C₁₋₄alkyl group, in particular an ethyl group;
- R¹ is attached in the para-position with respect to the bromine atom of formula II.

The compound of formula II is preferably 2-chloro-6-fluorobromobenzene or 2,4,6-trifluorobromobenzene, which can be prepared from commercially available 1,3,5-trifluorobenzene by known methods of bromination.

As a rule the reaction between the phenylbromide and the enolate obtained from the dialkyl malonate and the strong base is carried out at elevated temperatures between 30°C and 150°C, preferably between 35°C and 110°C, in particular between 50°C and 100°C, most preferred at the reflux temperature of the reaction medium.

The reaction mixture preferably is neutralized with dilute acid, the phases are separated and the organic layer is dried and concentrated. The crude product obtained can be purified according to standard methods for example by distillation in vacuo, chromatographic methods or crystallization.

However, the crude product obtained according to the process of this invention is pure enough to be used as intermediate without further purification.

The reaction is as a rule completed within 5 to 50 hours, in particular 10 to 25 hours.

In a particularly preferred embodiment of the process according to this invention diethyl malonate (2 to 3 moles) is added to a mixture of sodium hydride (1.5 to 2.5 moles) and 1,4-dioxane at 55 to 60°C within 2 to 5 hours. Subsequently copper(I) bromide, optionally complexed with dimethylsulphide, (0.1 to 0.3 moles) is added. A mixture of 2,4,6-trifluorobromobenzene (1 mol) and 1,4-dioxane is added. The reaction mixture is heated to 80-120°C for 10 to 20 hours. The reaction mixture is neutralized with a mineral acid in particular hydrochloric acid, and the organic phase is separated off and the aqueous phase is extracted. The combined organic phases are concentrated in vacuo. The residue is filtered, washed with an organic solvent and the solvent is distilled off. The residue is distilled in vacuo.

In order to facilitate a further understanding of the invention, the following illustrative examples are presented. The invention is not limited to the specific embodiments described or illustrated, but encompasses the full scope of the appended claims.

### Example 1

### Preparation of diethyl 2,4,6-trifluorophenylmalonate

Diethyl malonate (6.21 mol) is added to a mixture of sodium hydride (5.13 mol) and 1,4-dioxane (1400 ml) at 55 to 60 °C within 3 hours. The mixture is stirred for 10 minutes at 55 °C and copper(I) bromide (0.5 mol) is added. A mixture of 2,4,6-trifluorobromobenzene (2.50 mol) and 1,4-dioxane (600 ml) is added. The reaction mixture is heated at 100 °C for 14 hours and cooled to 15 °C. Hydrochloric acid (12N, 350 ml) is added slowly at 15 to 20 °C. The organic phase is separated off and the aqueous phase is extracted with ethyl acetate (250 ml) and toluene (200 ml). The combined organic phases are concentrated in vacuo. The residue is filtered over silica gel, washed with petroleum ether/ethyl acetate (15:1) and the solvent is distilled off. The residue is distilled in vacuo to yield 540 g of the product as a white solid.
Bp.: 88-105 °C at 0.1 mbar; mp.: 50 °C
Analogously are prepared
dimethyl 2,4,6-trifluorophenylmalonate,
diethyl 2,6-difluorophenylmalonate,
diethyl 2-chloro-6-fluorophenylmalonate,
diethyl 2,4,5-trifluorophenylmalonate, bp.: 100 °C at 0.006 mbar;
diethyl 2,4,6-trichlorophenylmalonate, bp.: 144-150 °C at 0.4 mbar; and
diethyl 2,3,4-trifluorophenylmalonate, bp.: 94 °C at 0.003 mbar;

### Examples 2 and 3

### Preparation of diethyl 2,4,6-trifluorophenylmalonate

Analogously to example 1 2,4,6-trifluorobromobenzene is treated with sodium diethylmalonate in different amounts.

The relative amounts of the reactants and solvents, the reaction temperature and yields are shown in table I in which the following abbreviations have been used:
- TFBB: 2,4,6-trifluorobromobenzene
- DMS: Dimethylsulphide
- cat.: Catalyst
- am_cat: amount of catalyst
- Time: reaction time
- DEM: diethyl malonate

**Table I**

| Examples 2 and 3 | | | | | | | |
|---|---|---|---|---|---|---|---|
| Example | cat | TFBB (mmol) | am_cat (mmol) | NaH (mmol) | DEM (mmol) | Time (hrs) | Yield (%) |
| 2 | CuBr/DMS | 23.7 | 28.9 | 57.8 | 57.8 | 14 | 68 |
| 3 | CuBr/DMS | 23.7 | 28.4 | 56.9 | 69.4 | 14 | 77 |

### Comparison Examples A to D

### Preparation of diethyl 2,4,6-trifluorophenylmalonate

Analogously to example 1 2,4,6-trifluorobromobenzene is treated with 1.22 equivalents of sodium diethylmalonate under different conditions.

The relative amounts of the reactants and solvents, the reaction temperature and yields are shown in table II.

**Table II**

| Comparison Examples A to D | | | | | | | |
|---|---|---|---|---|---|---|---|
| Example | cat | TFBB (mmol) | am_cat (mmol) | NaH (mol) | DEM (mol) | Time (hrs) | Yield (%) |
| A | CuBr/DMS | 23.7 | 28.9 | 28.9 | 28.9 | 6 | 35 |
| B | CuBr/DMS | 23.7 | 28.9 | 28.9 | 28.9 | 14 | 45 |
| C | CuBr | 23.7 | 28.9 | 28.9 | 28.9 | 14 | 41 |
| D | CuBr/DMS | 23.7 | 28.9 | 57.8 | 28.9 | 14 | traces |

## Claims

1. A process for the preparation of dialkyl phenylmalonates of formula I, 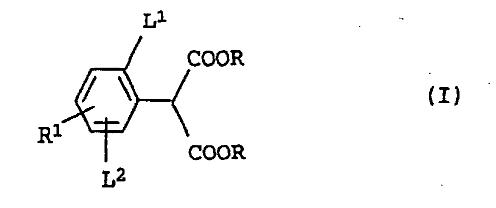 wherein
L¹ and L² each independently represent a fluoro or chloro atom;
R¹ represents a hydrogen or halogen atom or an C₁-C₁₀-alkyl or C₁-C₁₀-alkoxy group; and
R represents a C₁-C₄-alkyl group,
which comprises
treating 1.0 mole of a phenylbromide of formula II 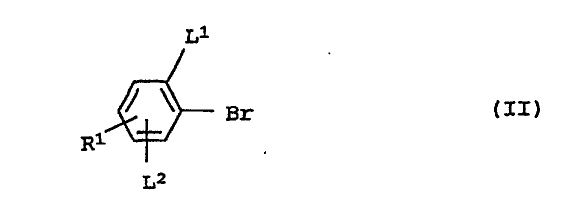 wherein
R¹, L¹ and L² have the meaning given for formula I, with 2.0 to 4.0 moles of a dialkyl malonate of formula III 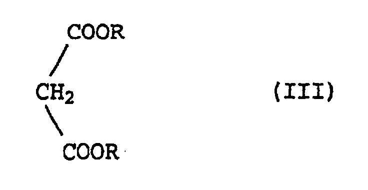
wherein
R has the meaning given for formula I, in an inert solvent in the presence of 2.0 to 3.8 moles of a base and 0.05 to 0.9 moles of copper-(I) bromide.

2. A process according to claim 1, wherein 0.05 to 0.3 moles of copper-(I) bromide are present.

3. A process according to claim 1, wherein the base is sodium hydride.

4. A process according to claim 1, wherein 1 mole of phenylpromide of formula II is treated with the enolate obtained from 2.5 to 3.5 moles of dialkyl malonate and 2.0 to 2.5 moles of the base.

5. A process according to claim 1, wherein a mixture consisting of the phenylbromide of formula II, the enolate obtained from the dialkyl malonate of formula III, the strong base, the copper-(I) bromide, optionally a complexing agent and an inert solvent is stirred at temperatures between 30°C and 150°C.

6. A process according to claim 4, wherein the inert solvent selected from the group consisting of diethylether, diisopropylether, tert-butylmethylether, 2,2-dimethoxypropane, diethoxyethane, tetrahydrofuran, tetrahydropyran and dioxane or a mixture of these solvents.

7. A process according to claim 1, wherein copper-(I) bromide is complexed by a dialkylsulphide.

8. A process according to claim 1, wherein R¹ is attached in the para-position with respect to the bromine atom of formula II.

9. A process according to claim 8, wherein the compound of formula II is 2,4,6-trifluorobromobenzene or 2-chloro-6-fluorobromobenzene.

## Patentansprüche

1. Verfahren zur Herstellung von Dialkylphenylmalonaten der Formel I, 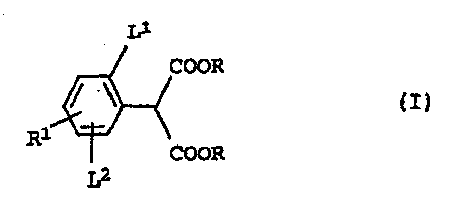 in der
L¹ und L² jeweils unabhängig ein Fluor- oder Chloratom bedeuten,
R¹ ein Wasserstoff- oder Halogenatom oder eine C₁-C₁₀-Alkyl- oder C₁-C₁₀-Alkoxygruppe bedeutet, und
R eine C₁-C₄-Alkylgruppe bedeutet,
**dadurch gekennzeichnet, daß** man
1,0 mol eines Phenylbromids der Formel II, 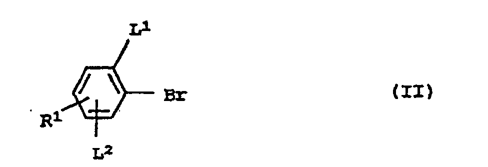 in der
R¹, L¹ und L² die für Formel I angegebene Bedeutung aufweisen, mit 2,0 bis 4,0 mol eines Dialkylmalonats der Formel III 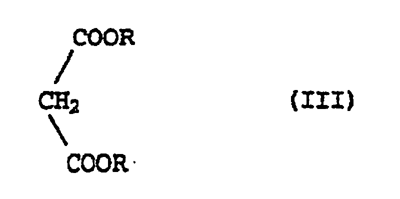
in der
R die für Formel I angegebene Bedeutung aufweist, in einem inerten Lösungsmittel in der Gegenwart von 2,0 bis 3,8 mol einer Base und 0,05 bis 0,9 mol Kupfer-(I)-bromid behandelt.

2. Verfahren nach Anspruch 1, **dadurch gekennzeichnet, daß** 0,05 bis 0,3 mol Kupfer-(I)-bromid vorliegen.

3. Verfahren nach Anspruch 1, **dadurch gekennzeichnet, daß** es sich bei der Base um Natriumhydrid handelt.

4. Verfahren nach Anspruch 1, **dadurch gekennzeichnet, daß** 1 mol Phenylbromid der Formel II mit dem aus 2,5 bis 3,5 mol Dialkylmalonat und 2,0 bis 2,5 mol der Base erhaltenen Enolat behandelt wird.

5. Verfahren nach Anspruch 1, **dadurch gekennzeichnet, daß** eine Mischung, die aus dem Phenylbromid der Formel II, dem aus dem Dialkylmalonat der Formel III erhaltenen Enolat, der starken Base, dem Kupfer-(I)-bromid, gegebenenfalls einem Komplexbildner und einem inerten Lösungsmittel besteht, bei Temperaturen zwischen 30°C und 150°C gerührt wird.

6. Verfahren nach Anspruch 4, **dadurch gekennzeichnet, daß** das inerte Lösungsmittel aus der Gruppe Diethylether, Diisopropylether, tert.-Butylmethylether, 2,2-Dimethoxypropan, Diethoxyethan, Tetrahydrofuran, Tetrahydropyran und Dioxan oder einer Mischung dieser Lösungsmittel stammt.

7. Verfahren nach Anspruch 1, **dadurch gekennzeichnet, daß** das Kupfer-(I)-bromid mit einem Dialkylsulfid komplexiert ist.

8. Verfahren nach Anspruch 1, **dadurch gekennzeichnet, daß** R¹ in para-Stellung in bezug auf das Bromatom der Formel II gebunden ist.

9. Verfahren nach Anspruch 8, **dadurch gekennzeichnet, daß** es sich bei der Verbindung der Formel II um 2,4,6-Trifluorbrombenzol oder 2-Chlor-6-fluorbrombenzol handelt.

## Revendications

1. Procédé pour la préparation de phényl-malonates dialkyliques répondant à la formule I 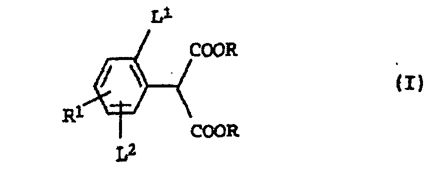 dans laquelle
L¹ et L², indépendamment l'un de l'autre, représentent un atome de fluor ou de chlore,
R¹ représente un atome d'hydrogène ou d'halogène, ou un groupe alkyle en C₁-C₁₀ ou alcoxy en C₁-C₁₀; et
R représente un groupe alkyle en C₁-C₄,
lequel comprend le traitement de 1,0 mole d'un bromure de phényle, répondant à la formule II 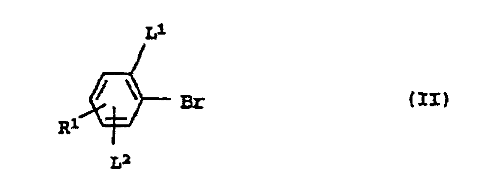 dans laquelle
R¹, L¹ et L² ont la signification donnée dans la formule I, avec 2,0 à 4,0 moles d'un malonate dialkylique, répondant à la formule III 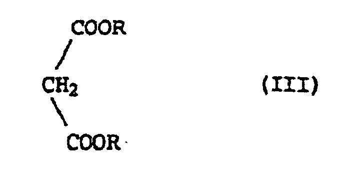
dans laquelle
R a la signification donnée dans la formule I, dans un solvant inerte, en présence de 2,0 à 3,8 moles d'une base et de 0,05 à 0,9 moles d'un bromure de cuivre (I).

2. Procédé selon la revendication 1, dans lequel 0,05 à 0,3 moles de bromure de cuivre (I) sont présentes.

3. Procédé selon la revendication 1, dans lequel la base est de l'hydrure de sodium.

4. Procédé selon la revendication 1, dans lequel 1 mole de bromure de phényle répondant à la formule II est traité avec l'énolate obtenu à partir de 2,5 à 3,5 moles de malonate dialkylique et de 2,0 à 2,5 moles de la base.

5. Procédé selon la revendication 1, dans lequel un mélange, comprenant le bromure de phényle répondant à la formule II, l'énolate obtenu à partir du malonate dialkylique répondant à la formule III, la base forte, le bromure de cuivre (I), éventuellement un agent complexant, et un solvant inerte, est agité à des températures comprises entre 30°C et 150°C.

6. Procédé selon la revendication 4, dans lequel le solvant inerte sélectionné parmi le groupe comprenant de l'éther diéthylique, de l'éther diisopropylique, de l'éther tert-butyfméthylique, du 2,2-diméthoxypropane, du diéthoxyéthane, du tétrahydrofurane, du tétrahydropyrane et du dioxane, ou un mélange de ces solvants.

7. Procédé selon la revendication 1, dans lequel le bromure de cuivre (I) est complexé avec un sulfure dialkylique.

8. Procédé selon la revendication 1, dans lequel R¹ est attaché en position para par rapport à l'atome de brome de la formule II.

9. Procédé selon la revendication 8, dans lequel le composé répondant à la formule II est le 2,4,6-trifluorobromobenzène ou le 2-chloro-6-fluorobromobenzène.
